# EUROPÄISCHE PATENTSCHRIFT

(11) **EP 1 893 176 B1**
(45) Veröffentlichungstag und Bekanntmachung des Hinweises auf die Patenterteilung: **02.01.2013**
(21) Anmeldenummer: 06763420.4
(22) Anmeldetag: 31.05.2006
(51) Int. Cl.: A61K 9/14, A61K 9/16, A61K 9/26, A61K 9/48, A61K 31/55, A61K 31/426, A61K 31/5415, A61K 31/4174, A61K 31/495, A61K 31/496, A61K 31/405, A61K 31/07

(54) **HERSTELLUNG VON FESTEN LÖSUNGEN SCHWERLÖSLICHER WIRKSTOFFE DURCH KURZZEITÜBERHITZUNG UND SCHNELLE TROCKNUNG**
PRODUCTION OF SOLID SOLUTIONS BASED ON POORLY-SOLUBLE ACTIVE SUBSTANCES BY A SHORT-TERM HEATING AND RAPID DRYING
PRODUCTION DE SOLUTIONS SOLIDES A BASE DE SUBSTANCES ACTIVES PEU SOLUBLES, PAR SURCHAUFFE DE COURTE DUREE ET SECHAGE RAPIDE

(30) Priorität: 09.06.2005 DE 102005026755
(43) Veröffentlichungstag der Anmeldung: 05.03.2008
(73) Patentinhaber: BASF SE, 67056 Ludwigshafen (DE)
(72) Erfinder: KOLTER, Karl, 67117 Limburgerhof (DE); SCHÖNHERR, Michael, 67227 Frankenthal (DE); ASCHERL, Hermann, 67246 Dirmstein (DE)
(86) Internationale Anmeldenummer: PCT/EP2006/062788
(87) Internationale Veröffentlichungsnummer: WO 2006/131481

(56) Entgegenhaltungen:
- WO-A-01/68092
- WO-A-99/56751
- DE-A1- 19 951 617
- US-A1- 2003 224 043
- ORIENTI I ET AL: "Polyvinylalcohol substituted with triethyleneglycolmonoethylether as a new material for preparation of solid dispersions of hydrophobic drugs" EUROPEAN JOURNAL OF PHARMACEUTICS AND BIOPHARMACEUTICS, ELSEVIER SCIENCE PUBLISHERS B.V., AMSTERDAM, NL, Bd. 54, Nr. 2, September 2002 (2002-09), Seiten 229-233, XP004377368 ISSN: 0939-6411 in der Anmeldung erwähnt
- CILURZO F ET AL: "Fast-dissolving mucoadhesive microparticulate delivery system containing piroxicam" EUROPEAN JOURNAL OF PHARMACEUTICAL SCIENCES, ELSEVIER, AMSTERDAM, NL, Bd. 24, Nr. 4, März 2005 (2005-03), Seiten 355-361, XP004759939 ISSN: 0928-0987
- LEUNER C ET AL: "Improving drug solubility for oral delivery using solid dispersions" EUROPEAN JOURNAL OF PHARMACEUTICS AND BIOPHARMACEUTICS, ELSEVIER SCIENCE PUBLISHERS B.V., AMSTERDAM, NL, Bd. 50, Nr. 1, 3. Juli 2000 (2000-07-03), Seiten 47-60, XP004257179 ISSN: 0939-6411

## Beschreibung

Die vorliegende Erfindung betrifft ein Verfahren zur Herstellung von festen Lösungen schwerlöslicher Wirkstoffe, pulverförmige Produkte die nach einem solchen Verfahren erhalten werden sowie deren Verwendung für Darreichungsformen.

In wässrigen Medien schwerlösliche Wirkstoffe bereiten in der Formulierung extreme Schwierigkeiten, da es kaum gelingt bioverfügbare Darreichungsformen zu entwickeln. Einer der vielversprechendsten Ansätze ist die Bildung fester Lösungen, bei denen der Wirkstoff in molekulardisperser Form eingearbeitet ist. Diese Form führt sehr oft zu deutlich höheren Bioverfügbarkeiten, da der Wirkstoff, nachdem sich das Polymer aufgelöst hat, in gelöster Form dem Körper oder der Pflanze angeboten wird.

In der Literatur wird der Begriff feste Lösung oft nicht korrekt gebraucht, da auch Einbettungen fester kristalliner Stoffe als feste Lösung bezeichnet werden. Strenggenommen handelt es sich hierbei aber um feste Dispersionen. In dieser Schrift wird unter fester Lösung eine echte molekulardisperse Verteilung verstanden.

Zur Herstellung fester Lösungen wurden schon verschiedene Stoffe insbesondere auch Polymere wie Polyvinylpyrrolidone, Celluloseester, Zucker, Zuckeralkohole, Stärken und natürliche Polysaccharide beschrieben, die geeignet sein sollen.

Die Herstellung solcher festen Lösungen ist bis heute ein recht aufwändiger Prozess.

Folgende Methoden stehen zur Verfügung:
1. Schmelzen von Wirkstoff und Polymer bei hoher Temperatur und Extrusion. Dieses Verfahren hat den Nachteil, dass minutenlang hohe Temperaturen auf den Wirkstoff einwirken und zudem große Formkörper entstehen, die mühsam durch Mahlung zerkleinert werden müssen, um tablettierbar zu sein. Außerdem besitzen die Formkörper im allgemeinen keine Porosität, so dass die Verpressbarkeit sehr schlecht ist. Die Tabletten weisen häufig eine niedrige Bruchfestigkeit und eine hohe Friabilität auf. Nur Produkte mit einem gewissen Mindestmaß an Porosität lassen sich gut verpressen. Hinzu kommt, dass bei der Schmelzextrusion neben der Temperaturbelastung auch Scherkräfte einwirken, die zur Zersetzung der Wirkstoffe führen können.
2. Lösen von Wirkstoff und Polymer in einem organischen Lösungsmittel, das beide löst, und Abdampfen des Lösungsmittels oder Sprühtrocknen. Dieses Verfahren hat per se den Nachteil, dass in grossem Umfang organische Lösungsmittel eingesetzt werden müssen, die umweltschädlich und explosionsgefährlich sind, und deren Verwendung erheblich Kosten verursacht. Außerdem hat man oft Schwierigkeiten ein Lösungsmittel zu finden, das sowohl das hydrophile Polymer wie auch den lipophilen Arzneistoff löst.
3. Dispergieren von Wirkstoff in einer wässrigen Polymerlösung, zum Beispiel durch Nassmahlung und Sprühtrocknen. Hierbei werden, wenn der Wirkstoff nicht wasserlöslich ist, keine festen Lösungen gebildet, sondern nur feste Dispersionen, die bei weitem nicht über die Eigenschaften verfügen wie molekulardisperse Lösungen, insbesondere nicht im Hinblick auf die Bioverfügbarkeit.

In der US 5876760 werden sprühgetrocknete Pulver aus Pranlukast, Sacchariden, gegebenenfalls wasserlöslichen Polymeren und Surfactants beschrieben. Der Wirkstoff ist in einer wässrigen Lösung der Hilfsstoffe suspendiert und diese wird bei üblichen Bedingungen sprühgetrocknet: Im Endprodukt liegt der Wirkstoff in kristalliner Form vor.

In der US 6197781 wird eine Rapamycin enthaltende Formulierung beschrieben, wobei der Wirkstoff in einem Lösungsmittel gelöst wird und zusammen mit einemTräger, der aus Polyoxyethylen-polyoxypropylen Blockcopolymer, Polyvinylpyrrolidon, mikrokristalline Cellulose und einer wasserlöslichen Saccharose bestehen kann, bei Temperaturen von 20 -80° C sprühgetrocknet wird. Es werden dabei große Anteile organischer Lösungsmittel verwendet und die Temperaturen der Lösung vor der Sprühtrocknung liegen deutlich unter 100°C.

In der WO 99/56751 werden amorphe Paroxetin Formulierungen beschrieben, die durch Mischen von Paroxetin-Salzen, vorzugsweise dem Hydrochlorid, mit Wasser und einem Polymer und anschließendes Trocknen bei 25 - 100°C , vorzugsweise bei 60°C, hergestellt werden. Die Paroxetinsalze sind in Wasser schon bei niedrigen Temperaturen löslich, so dass die Herstellung einer festen Lösung kein besonderes Problem darstellt. Die WO 01/30349 bezieht sich ebenfalls auf die Verarbeitung von amorphen Paroxetinsalzen in Polyvinylpyrrolidon und einer zusätzlichen Säure. Die Herstellung erfolgt bei Temperaturen von 15 - 40°C.

Die WO 03/000294 bezieht sich auf feste Dispersionen von schlecht wasserlöslichen Arzneistoffen in einer Matrix mit einem löslichkeitsverbessernden Polymer. Beschrieben ist beispielsweise die Herstellung unter Anwendung von organischen Lösungsmitteln oder durch Verarbeitung in der Schmelze.

Pharmazeutische Darreichungsformen mit einem Wirkstoff in zwei verschiedenen physikalischen Formen werden in DE 19951617 behandelt. Dabei liegt der Wirkstoff teilweise in partikulärer Form und in gelöster Form vor. Die Herstellung kann auf an sich übliche Weise unter Einsatz von organischen Lösungsmittein erfolgen. Vorzugsweise erfolgt die Herstellung der festen Lösung über Schmelzextrusion.

US 2001/00076788 und US 2003/0082239 beschreiben feste Dispersionen von Itraconazol mit wasserlöslichen Polymeren, wobei die Herstellung durch Schmelzextrusion und anschließende Mahlung erfolgt. Diese Zubereitungen besitzen die schon bekannten Nachteile wie z. B. schlechte Tablettierbarkeit.

Zur Herstellung der in US 2002/0009494 beschriebenen festen Dispersionen wird ein schwerlöslicher Arzneistoff zusammen mit Hydroxypropylmethylcelluloseacetatsuccinat in einem organischen Lösungsmittel gelöst und sprühggetrocknet. Zur Herstellung von einem Teil Zubereitung werden ungewöhnlich große Mengen an Lösungsmittel benötigt.

Gemäß der US 2002/0031547 werden Arzneistoffe mit einem gelbildenden wasserlöslichen Polymer in einem organischen Lösungsmittel gelöst und getrocknet. Anschließend wird diese Zubereitung mit einem Salz aus Alkali und einer schwachen oder starken Säure gemischt und tablettiert. Bei den gelbildenden Polymeren handelt es sich um Celluloseether. Da gelbildende Polymere den Zerfall und die Wirkstofffreisetzung aus Tabletten verlangsamen, ist nicht verwunderlich, dass zur Zerfallsbeschleunigung weitere Zusatzstoffe erforderlich sind.

Die WO 01/47492 beschreibt feste Dispersionen von praktisch unlöslichen Arzneistoffen mit Polymeren, die saure funktionelle Gruppen besitzen. Der Arzneistoff liegt partikulär vor und zur Herstellung werden organische Lösungsmittel, insbesondere Methylenchlorid, verwendet.

Ebenso beschreibt auch die WO 2002051385 die Herstellung von festen Lösungen unter Verwendung von organischen Lösungsmitteln. Als Polymere werden Cellulosederivate und Polyvinylpyrrolidon eingesetzt. Weitere Hilfsstoffe sind Benetzungsmittel.

Weitere feste Lösungen unter Anwendung von organischen Lösungsmitteln werden in WO 2001068092, EP 1027886, EP 1027887, EP 1027888, US 2001/0053778, WO 03/000235 , WO 2001068092, WO 2003000226, US 2001/0053791 beschrieben.

In der US 2003/0104068 und der US 2003/082236 werden Einbettungen von Wirkstoffteilchen, die eine Größe kleiner 2 µm besitzen, offenbart. Aufgrund des partikulären Zustandes handelt es sich nicht um eine feste Lösung im eigentlichen Sinn.

In der DE 4329446 wird ein Verfahren beschrieben, bei dem eine Schmelzemulsion eines Wirkstoffes in Wasser oder Mischungen von Wasser mit organischen Lösungsmitteln oberhalb des Schmelzpunktes des Wirkstoffes in Gegenwart eines Schutzkolloides hergestellt und diese sprühgetrocknet wird. Dabei entstehen kolloidale Wirkstoffpartikel und keine festen Lösungen.

Aufgabe der vorliegenden Erfindung war es, ein Verfahren zu finden, das organische Lösungsmittel vermeidet, keine große Temperaturbelastung für die Wirkstoffe mit sich bringt, direkt ein Produkt mit guter Tablettierbarkeit und Fließfähigkeit liefert und einfach durchzuführen ist.

Demgemäß wurde ein Verfahren zur Herstellung von pulverförmigen festen Lösungen schwerlöslicher Substanzen, in denen die schwerlösliche Substanz molekulardispers in einer Hilfsstoffmatrix vorliegt, durch Zerstäuben einer Lösung der schwerlöslichen Substanz und der Matrixhilfsstoffe gefunden, welches dadurch gekennzeichnet ist, dass eine wässrige Suspension der schwerlöslichen Substanz in Gegenwart der Matrixhilfsstoffe bei Drücken von 0,08 bis 20 MPa auf Temperaturen > 90°C bis 350 °C erhitzt und die schwerlösliche Substanz in Lösung gebracht wird, und anschließend durch Zerstäuben und Trocknen in pulverförmige Form überführt wird, wobei die Temperatur der Sprühlösung bei Zuführung in die Zerstäubungsvorrichtung > 90 bis 350 °C beträgt.

Als feste Lösung wird erfindungsgemäß ein Zustand bezeichnet, bei dem der Wirkstoff molekulardispers verteilt in einer Matrix aus Hilfsstoffen vorliegt. In diesem Zustand sind röntgendiffraktometrisch keine kristallinen Anteile des Wirkstoffs mehr festzustellen. Da die Nachweisgrenze für kristalline Anteile bei der Röntgendiffraktometrie bei 3 Gew.-% liegt, bedeutet der Ausdruck "keine kristallinen Anteile",dass weniger als 3 Gew.- % kristalline Anteile vorhanden sind. Der Zustand der molekulardispersen Verteilung kann mit Hilfe der Methode der Differential Scanning Calorimetry (DSC) ermittelt werden. Bei einer molekulardispersen Verteilung ist im Bereich des Schmelzpunkts des Wirkstoffs kein Schmelzpeak mehr zu beobachten. Die Nachweisgrenze dieser Methode liegt bei 1 Gew.- %.

Als schwerlösliche Substanzen im Sinne der Erfindung sind Stoffe zu verstehen, deren Sättigungslöslichkeit bei Raumtemperatur (20°C) in mindestens einem der folgenden Medien kleiner als 1 Gew.-% ist: Wasser, 0,1 molare wässrige Salzsäure, wässriger Phosphatpuffer pH 7,2, 0,9 gew.-%ige wässrige Kochsalzlösung.

Als schwerlösliche Substanzen kommen erfindungsgemäß eine Vielzahl von Wirk- und Effektstoffen in Betracht, insbesondere pharmazeutische oder kosmetische Wirkstoffe, Wirkstoffe für Nahrungsergänzungsmittel oder dietätische Mittel oder Lebensmittelzusätze.

Schwerlösliche Substanzen im Sinne der Erfindung sind beispielsweise:
Piroxicam, Clotrimazol, Carbamazepin, 17-Beta-Estradiol, Sulfathiazol, Fenofibrat, Benzocain, Lidocain, Dimethinden, Biperiden, Bisacodyl, Clioquinol, Droperidol, Haloperidol, Nifedipin, Nitrendipin,Tetracyclin, Phenytoin, Glafenin, Floctafenin, Indometacin, Ketoprofen, Ibuprofen, Dipyridamol, Mefenaminsäure, Amiodaron, Felodipin, Itraconazol, Ketoconazol, Danazol, Furosemid, Tolbutamid, Ritonavir, Lopinavir, Naproxen, Spironolacton, Propafenon, Progesteron, Paclitaxel, Docetaxel, Theophyllin, Hydrocortison, Betacarotin, Vitamin A, Tocopherolacetat, Riboflavin, Vitamin Q 10, Vitamin D, Vitamin K, Disulfiram, Nimodipin, Chlorthiazid, Chlorpropamid, Dicoumarol, Chloramphenicol, Digoxin, Lonidamine, Pizotifen, Atovaquone, Amprenavir, Bexaroten, Calcitrol, Clofazimin, Doxercalciferol, Dronabinol, Durasteride, Etoposid, Loratadin, Risperidon, Saquinavir, Sirolimus, Valproinsäure, Amphotericin, Alprostadil, Carmustin, Chlordiazepoxid, Fenoldopam, Melphalan, Methocarbamil, Oxytetracyclin, Docetaxel, Fulvestrant, Propofol, Voriconazol, Ziprasidon, Leuprolidacetat, Viadur, Valrubicin, Tramadol, Celecoxib, Etodolac, Refocoxib, Oxaprozin, Leflunomide, Diclofenac, Nabumetone, Ibuprofen, Flurbiprofen, Tetrahydrocannabinol, Capsaicin, Ketorolac, Albendazol, Ivermectin, Amiodaron, Zileuton, Zafirlukast, Albuterol, Montelukast, A-zithromycin, Ciprofloxacin, Clarithromycin, Dirithromycin, Rifabutin, Rifapentin, Trovafloxacin, Baclofen, Ritanovir, Saquinavir, Nelfinavir, Efavirenz, Dicoumarol, Tirofibran, Cilostazol,Ticlidopin, Clopidrogel, Oprevelkin, Paroxetin, Sertralin,Venlafaxin, Bupropion, Clomipramin, Miglitol, Repaglinid, Glymeprid, Pioglitazon, Rosiglitazon, Troglitazon, Glyburid, Glipizid, Glibenclamid" Fosphenytion, Tiagabin, Topiramat, Lamotrigin, Vigabatrin, Amphotericin B, Butenafin, Terbinafin, Itraconazol, Flucanazol, Miconazol, Ketoconazol, Metronidazol, Griseofulvin, Nitrofurantoin, Lisinopril, Benezepril, Nifedipin, Nilsolidipin, Telmisartan, Irbesartan, Eposartan, Valsartan, Candesartan, Minoxidil, Terazosin, Halofantrine, Mefloquin, Dihydroergotamin, Ergotamin, Frovatriptan, Pizotifen, Sumatriptan, Zolmitriptan, Naratiptan, Rizatriptan, Aminogluthemid, Busulphan, Cyclosporin, Mitoxantron, Irinotecan, Etoposid, Teniposid, Paclitaxel, Tacrolimus, Sirolimius, Tamoxifen, Camptothecan, Topotecan, Nilutanid, Bicalutanid, Toremifen, Atovaquon, Metronidazol, Furazolidon, Paricalcitol, Benzonatat, Midazolam, Zolpidem, Gabapentin, Zopiclon, Digoxin, Beclomethason, Budesonide, Betamethason, Prednisolon, Cisapride, Cimetidin, Loperamid, Famotidin, Lanosprazol, Rabeprazol, Nizatidin, Omeprazol, Citrizin, Cinnarizin, Dexchlopheniramin, Loratadine, Clemastine, Fexofenadine, Chlorpheniramine, Acutretin, Tazaroten, Cal-Ciprotien, Calcitriol, Targretin, Ergocalciferol, Cholecalciferol, Isotretinoin, Tretinoin, Calcifediol, Fenofibrat, Probucol, Gemfibrozil, Cerivistatin, Pravastatin, Simvastatin, Fluvastatin, Atorvastatin, Tizanidin, Dantrolen, Isosorbiddinatrat, Dihydrotachysterol, essentielle Fettsäuren, Codein, Fentanyl, Methadon, Nalbuphin, Pentazocin, Clomiphen, Danazol, Dihydroepiandrosteron, Medroxyprogesteron, Progesteron, Rimexo-Ion, Megesterolacetate, Oestradiol, Finasterid, Mefepriston, L-Thryroxine, Tamsulosin, Methoxsalen, Tacrin, Donepezil, Raloxifen, Vertoporfin, Sibutramin, Pyridostigmin, sowie deren Isomere, Derivate, Salze oder Mischungen.

Die nach dem erfindungsgemäßen Verfahren hergestellten festen Lösungen können die folgende mengenmäßige Zusammensetzung aufweisen :
(i) 1 bis 50 Gew.-% mindestens eines Wirkstoffs,
(ii) 10 bis 99 Gew.-% mindestens eines wasserlöslichen Matrixhilfsstoffs
(iii) 0 bis 30 Gew.-% eines oder mehrerer Tenside,
(iv) 0 bis 30 Gew.-% Co-solubilisatoren,
(v) 0 bis 50 Gew.-% weiterer Hilfsstoffe,
wobei sich die Mengen der Komponenten (i) bis (v) zu 100 Gew.-% addieren.

Als matrixaufbauende Hilfsstoffe sind grundsätzlich alle Stoffe geeignet, die in der Lage sind, mit Wirkstoffen feste Lösungen zu bilden.

Geeignet sind beispielsweise wasserlösliche Polymere aus folgenden Strukturklassen:
Polyvinylpyrrolidone, Vinylpyrrolidon-Vinylacetat-Copolymere, Polyvinylcaprolactame, Polyvinylformamid, Polyvinylacetamid, Polyacrylate, Polymethacrylate, Polyacrylamide, Polyethylenimine, Polyvinylamine
Hydroxyalkylcellulosen, Alkyl-hydroxyalkylcellulosen, Carboxyalkylcellulosen, Alkylhydroxyalkylcelluloseacetatsuccinate, Alkyl-hydroxyalkylcelluloseacetatphthalate, Alkylhydroxyalkylcellulosephthalate, Celluloseacetatphthalate
Stärken, Hydroxyalkylstärken, Carboxyalkylstärken, modifizierte Stärke, Octenylsuccinatstärken,
Dextrane,
Polyoxyethylen-polyoxypropylen-Blockcopolomere,
Polyethylenoxide, Polypropylenoxide,
Polyaminosäuren

Aber auch niedermolekulare Trägerstoffe können eingesetzt werden:
Zucker wie Saccharose, Glucose, Maltose, Xylose, Fructose, Ribose, Arabinose, Galactose, Trehalose
Zuckeralkohole wie Sorbit, Mannit, Xylit, Erythritol, Palatinit, Maltitol, Lactitol
Harnstoff
Nicotinamid
Aminosäuren
Cyclodextrine

Bevorzugt sind Stoffe, die über Amidstrukturen verfügen, da diese hohe Konzentrationen an Wirkstoffen zu lösen vermögen.

Als polymere Matrixhilfsstoffe werden bevorzugt Polyvinylpyrrolidon, Copolymere aus N-Vinylpyrrolidon und Vinylacetat verwendet. Weitrhin bevorzugt ist auch die verwendung von Alkylmethacrylaten oder Alkylacrylaten.

Die K-Werte nach Fikentscher der Polymere in einer 1 gew.-%igen wässrigen Lösungkönnen 5 bis 120, bevorzugt 10 bis 95, betragen.

Als niedermolekulare Matrixhilfsstoffe eignet sich insbesondere Harnstoff.

Die Matrixhiffsstoffe (ii) werden vorzugsweise in Mengen von 30 bis 90 Gew.-% eingesetzt.

Zur weiteren Verbesserung der Löslichkeit können zusätzlich Solubilisatoren eingesetzt werden. Als Solubilisatoren eignen sich Tenside, die in der Regel einen HLB-Wert größer 10 aufweisen (HLB: Hydrophilic Lipophilic Balance). Solche Solubilisatoren sind beschrieben in: Fiedler, Lexikon der Hilfsstoffe, Editio Cantor Verlag Aulendorf, 5. Auflage, Seite 117-121

Besonders bewährt haben sich:
Alkali- oder Ammoniumsalze von Fettsäuren, Alkali- oder Ammoniumsalze von sulfonierten oder sulfatierten Fettsäuren, Polyoxyethylenfettsäureester, Polyoxyethylenfettolkohole, Polyoxyethylenglycerinfettsäureester,

Polyoxyethylenglycerinfettolkohole, Polyoxyethylensorbitanfettsäureester, Ethoxyliertes Ricinusöl, Ethoxyliertes hydriertes Ricinusöl, ethoxylierte 12-Hydroxystearinsäure, Poloxamere oder Mischungen davon.

Solche Tenside (iii) werden vorzugsweise in Mengen von 1 bis 20 Gew.-% eingesetzt.

In besonderen Fällen hat sich auch die Verwendung von Cosolubilisatoren (iv) mit einem HLB-Wert kleiner 10 als vorteilhaft erwiesen, weil dadurch die Bildung und die Stabilität der festen Lösung gefördert werden. Diese Stoffe sind ebenfalls beschrieben in: Fiedler, Lexikon der Hilfsstoffe, Editio Cantor Verlag Aulendorf, 5. Auflage, Seite 115-117

Beispielsweise können folgende Stoffe eingesetzt werden:
Polyoxyethylenfettsäureester, Polyoxyethylenfettolkohole, Polyoxyethylenglycerinfettsäureester, Polyoxyethylenglycerinfettolkohole,
Glycerinfettsäureester, Glycerinfettolkohole, Sorbitanfettsäureester

Es kann sich auch empfehlen, als zusätzliche Lösungsvermittler organische Lösungmittel in Mengen von bis zu 10 Gew.-% einzusetzen. Als organische Lösungsmittel kommen Ethanol, Isopropanol oder Aceton in Betracht. Vorzugsweise wird jedoch auf den Einsatz von organischen Lösungsmitteln verzichtet.

Zum Teil haben die Wirkstoffe, Solubilisatoren und Cosolubilisatoren eine erhebliche weichmachende Wirkung, d. h. sie senken die Glasübergangstemperatur des Polymers deutlich, wodurch die Sprühtrocknung mitunter schwierig wird. In diesen Fällen hat sich die Verwendung eines Adsorbens als sehr vorteilhaft erwiesen. Dieses Adsorbens saugt die flüssige oder halbfeste Wirkstoff-Polymerlösung auf erzeugt damit eine feste Zubereitung, die gut anwendbar ist. Als Adsorbentien können z. B. folgende Stoffe eingesetzt werden: Kieselsäure, hydrophobe Kieselsäure, Alkali- oder Erdalkalisilikate, Erdalkali-Aluminiumsilikate, quervernetztes Polyvinylpyrrolidon, Cellulose, Stärke, quervernetzte Natriumcarboxymethylstärke, quervernetzte Natriumcarboxymethylcellulose.

Das Adsorbens wird in der Regel vor dem Erwärmungsschritt in der Sprühlösung suspendiert und mitgetrocknet. Ein Teil kann aber auch pulverförmig in den Sprühturm eingeblasen werden.

Darüber hinaus können, um spezifische Charakteristika zu erzielen, weitere Hilfsstoffe (v) Verwendung finden:
Weichmacher
Antioxidantien
Konservierungsmittel
Farbstoffe
Aromen und Geruchstoffe
Füllstoffe,
Antiklebemittel
Zerfallsfördernde Hilfsstoffe. (Sprengmittel)
Retardierungsmittel
Solche Hilfsstoffe sind vorzugsweise in Mengen von 0,1 bis 20 Gew.-% enthalten.

Erfindungsgemäß werden zunächst durch Erhitzen wässrige Lösungen, enthaltend den Wirkstoff und die Matrixhilfsstoffe sowie gegebenenfalls die weiteren Komponenten (iii) bis (v), hergestellt. Vorzugsweise wird Wasser als alleiniges Lösungsmittel verwendet.

Grundsätzlich gibt es folgende Methoden zur Herstellung der Lösungen:
Methode A: Man stellt eine wässrige Suspension her, die den Wirkstoff in suspendierter Form und die Matrixhilfsstoffe in gelöster Form enthält sowie gegebenenfalls die weiteren Komponenten. Dazu kann man entweder zunächst die Matrixhilfsstoffe in Wasser lösen und den Wirkstoff in dieser Lösung suspendieren oder eine wässrige Suspension des Wirkstoffs mit den Matrixhilfsstoffen versetzen. Die so erhaltene Suspension wird dann in einer geeigneten Vorrichtung bis zur Lösung des Wirkstoffs erhitzt.
Methode B: Man stellt wie unter Methode A beschrieben eine wässrige Suspension des Wirkstoffs, die die Matrixhilfsstoffe in gelöster Form enthält, her und erhitzt diese durch Vermischen mit einem heissen Wasserstrom oder einem Wasserdampfstrom bis zur Lösung des Wirkstoffs.
Methode C: In einer geringfügigen Abwandlung der Methode B kann man auch Matrixhilfsstoffe, sofern sie temperaturstabil sind, in einem heißen Wasserstrom lösen und mit einer Suspension des Wirkstoffes in Wasser vermischen.

Unabhängig von der gewählten Methode gilt Folgendes:

Für die Dispergierung des Wirkstoffes in Wasser oder der wässrigen Polymerlösung ist eine kleine Korngröße von Vorteil, weil diese erstens die Dispergierung erleichtert und zweitens der Auflöseprozess in der Hitze schneller erfolgt. Falls ein grober Wirkstoff gesetzt wird, kann dieser auch in der Polymerlösung zerkleinert oder gemahlen werden, bevor die Suspension erhitzt wird. Für die Zerkleinerung können beispielsweise Hochdruckhomogenisatoren, Rotor-Stator-geräte, Kugelmühlen oder Kolloidmühlen verwendet werden. Prinzipiell kann der Wirkstoff aber auch wie beschrieben zuerst in Wasser gegeben werden und anschließend erst das Polymer zugesetzt werden.

Das Erhitzen der wässrigen Suspension erfolgt kontinuierlich in einer geeigneten Vorrichtung.

Die Erhitzung kann beispielsweise in jedem geeigneten Wärmeaustauscher erfolgen, wobei man als Wärmeaustauscher allgemein Vorrichtungen bezeichnet, in welchen durch ein Wärmeübertragungsmittel Wärme auf ein anderes Medium transportiert wird, um eine Erhitzung zu erzielen.

Beim mittelbaren Wärmeaustausch sind Wärmeübertragungsmedium und zu erhitzendes Medium durch Wärmeaustauschflächen getrennt. Als Wärmeübertragungsmedium eignen sich heisses Öl, heisser Dampf oder überhitztes Wasser oder auch allgemein heisse Gase oder heiße Flüssigkeiten. Das Wärmeübertragungsmedium kann im Gegenstrom zu der zu erhitzenden wässrigen Suspension geleitet werden. Weiterhin kann auch das zu erhitzende Medium kontinuierlich durch ein statisches Wärmeübertragungsmedium geleitet werden.

Beim unmittelbaren Wärmeaustausch, wie er erfindungsgemäß nach den Methoden B) oder C) erfolgt, berühren sich beide Medien. Als unmittelbare Wärmeaustauschmittel kommen daher überhitztes Wasser oder Wasserdampf als Wärmeübertragungsmittel in Betracht.

Generell kann die Erhitzung der Wirkstoffsuspension mit allen Verfahren erfolgen, die eine sehr schnelle Heizrate ermöglichen. So sind auch elektrische, induktive oder Mikrowellen-Enivärmungen möglich.

Um den Wirkstoff in Wasser in Lösung zu bringen, wird die wässrige Suspension auf Temperaturen erhitzt, die über der Siedetemperatur der Mischung bei Normaldruck liegen, erhitzt. Dabei können Temperaturen von > 90 °C bis 350 °C, vorzugsweise 110 bis 300 °C, besonders bevorzugt 120 bis 250 °C, gewählt werden.

Um eine thermische Belastung der Einsatzstoffe zu vermeiden, werden unabhängig davon, welche der geschilderten Methoden angewandt wird, die Verweilzeiten beim Erhitzen auf > 90 °C im Bereich von Sekunden gehalten. Vorzugsweise beträgt die Verweilzeit des wirkstoffhaltigen Mediums in der zum Erhitzen eingesetzten Vorrichtung kleiner 180 Sekunden, besonders bevorzugt kleiner 60 Sekunden, ganz besonders bevorzugt kleiner 15 Sekunden. Um eine vollständige Lösung des Wirkstoffs zu erzielen, wird im allgemeinen eine Mindestverweilzeit von 0,5 Sekunden gewählt.

Üblicherweise beträgt der Feststoffgehalt der Lösungen 1 bis 70 Gew.-%, bevorzugt 3 bis 60 Gew.-%, besonders bevorzugt 5 bis 40 Gew.-%.

Die heiße und unter Druck stehende wässrige Lösung von Wirkstoff, Matrixhilfsstoffen und gegebenenfalls weiteren Komponenten wird nach Passieren der Vorrichtung direkt in eine Zerstäubungsvorrichtung geleitet. Das Zerstäuben kann über Düsen erfolgen, wobei sich grundsätzlich Ein- oder Mehrstoffdüsen eignen, oder über rotierende Scheiben erfolgen. Die Zerstäubung der Zubereitung im Trocknungsturm erfolgt vorzugsweise über Einstoffdüsen bei Drücken von 10 bis 250 bar. Es können aber auch Mehrstoffdüsen, insbesondere Zweistoffdüsen, eingesetzt werden, wobei der Druck des Zerstäubungsgases 0,15 bis 10 MPa betragen kann.

Die Turmeingangstemperaturen des Trocknungsgases, liegen zwischen 50 und 200°C vorzugsweise zwischen 70 und 180°C. Als Trocknungsgase eignen sich Luft oder inerte Gase wie Stickstoff, Argon oder Helium. Die Turmausgangstemperaturen liegen bei 40 bis 120 °C. Das Trocknungsgas kann im Gleichstrom oder im Gegenstrom zu den Flüssigkeitströpfchen in den Trockenturm eingeleitet werden, vorzugsweise im Gleichstrom.

Neben der einfachen Sprühtrocknung kann auch eine agglomerierende Sprühtrocknung mit internem und/oder externem Wirbelbett (z.B. FSD-Technologie der Firma Niro)durchgeführt werden, wobei die bei der Sprühtrocknung gebildeten Teilchen zu größeren Gebilden agglomeriert werden.

Generell können alle Trocknungstechniken, bei denen eine Lösung zerstäubt wird, eingesetzt werden, so auch die Wirbelschicht-Sprühgranulation.

Sofern die sprühgetrockneten Teilchen eine gewisse Klebetendenz aufweisen, bietet sich die Bepuderung mit einem sehr feinteiligen Feststoff an. Dabei wird dieser feinteilige Feststoff in den Sprühturm eingestäubt und sorgt so dafür dass keine Verklebung oder Verklumpung auftritt. Hochdisperse Kieselsäure hat sich hier gut bewährt. Jedoch können auch andere Stoffe eingesetzt werden, beispielsweise hydrophobe Kieselsäure, alkali- oder Erdalkalisilikate, Erdalkali-Aluminiumsilikate, quervernetztes Polyvinylpyrrolidon, Cellulose, Stärke, quervernetzte Natriumcarboxymethylstärke oder quervernetzte Natriumcarboxymethylceliulose.

Gemäß einer Ausführungsform zur erfindungsgemässen Herstellung der festen Lösungen nach Methode A wird der Arzneistoff demgemäß in einer wässrigen Lösung des Polymers dispergiert und die Suspension in einer geeigneten Vorrichtung auf Temperaturen oberhalb von 90° erhitzt, so dass die Wirkstoffkristalle in Lösung gehen. Die Erhitzung der wirkstoffhaltigen Polymerlösung sollte möglichst schnell erfolgen, um die Temperaturbelastung des Arzneistoffes gering zu halten. Dazu wird die wirkstoffhaltige Suspension kontinuierlich durch eine geeignete Vorrichtung geleitet, wobei die Verweilzeiten wie geschildert vorzugsweise im Bereich von wenigen Sekunden liegen. Diese erhitzte unter Druck stehende Wirkstofflösung wird im Anschluss daran zerstäubt und getrocknet. Die Temperatur der Sprühlösung kurz vor der Zerstäubung, also vor Einleitung in die Zerstäubungsvorrichtung, beträgt >90 - 350°C, vorzugsweise 110 - 300°C und besonders bevorzugt 120 - 250°C. Der Druck der Sprühlösung beträgt dabei 0,08 bis 20 MPa, vorzugsweise 1 bis 15 MPa.

Gemäß einer bevorzugten Ausführungsform der Erfindung kann die wirkstoffhaltige Polymerlösung durch eine dünne Rohrleitung gepumpt werden, die sich in einem heißen Ölbad befindet, das Temperaturen von 110 - 500°C, vorzugsweise 130 - 300°C aufweist. Dadurch ist ein schneller Wärmeübergang möglich. Die Einstellung der Temperatur der wirkstoffhaltigen Polymerlösung erfolgt durch Variation der Ölbadtemperatur und der Durchflussgeschwindigkeit. Direkt im Anschluss an die Durchleitung durch die Rohrleitung wird die heisse unter Druck stehende Lösung über eine Sprühdüse zerstäubt und mit warmem Trocknungsgas getrocknet. Durch die Verdampfung des Wassers erfolgt eine schlagartige Abkühlung und Trocknung der Sprühtröpfchen.

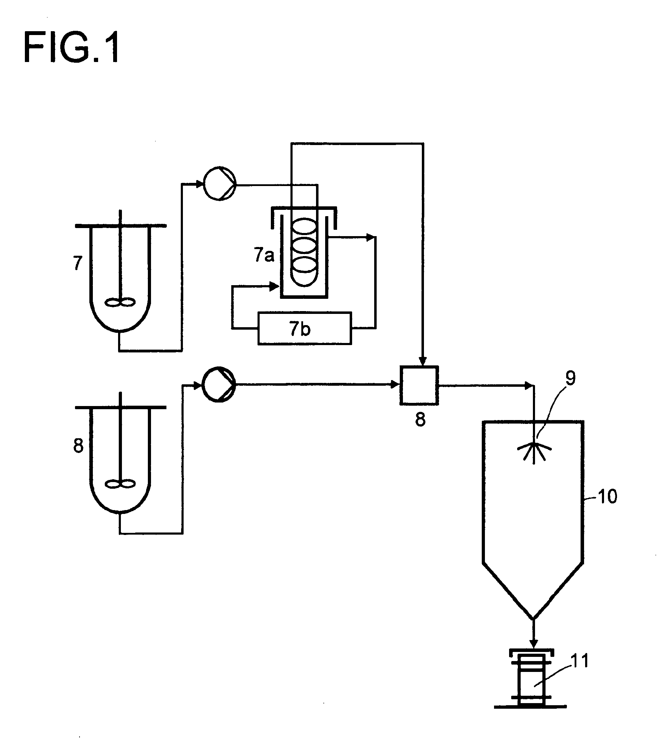

Eine solche Verfahrensweise ist beispielsweise in Abbildung 1 schematisch dargestellt. Dabei wird in einem mit einem Rührer ausgestatteten Behälter 1 die Suspension des Wirkstoffs in der wässrigen Lösung der Matrixhilfsstoffe hergestellt, die Suspension anschliessend kontinuierlich in einer Rohrschlange durch einen Wärmetauscher 2 gepumpt, der mit einer Heizung 2a zur Erhitzen des Wärmeübertragungsmediums ausgestattet ist, und die Lösung anschliessend über eine Düse 3 in einem Sprühturm 4 zerstäubt und getrocknet, und die entstehende teilchenförmige feste Lösung 5 aufgefangen.

Gemäss einer weiteren Ausführungsform der Erfindung kann die im Folgenden geschilderte Vorgehensweise nach Methode B gewählt werden. Diese Vorgehensweise empfiehlt sich besonders, wenn die Temperaturbelastung der schwerlöslichen Substanz weiter minimiert werden soll. Die Suspendierung der schwerlöslichen Substanz in der Polymerlösung erfolgt bei Raumtemperatur oder leicht erhöhter Temperatur, bei der sich der Wirkstoff noch nicht zersetzt. Diese Suspension wird einer Mischzelle zugeführt, in der sie mit überhitztem Wasser oder Wasserdampf turbulent gemischt wird. Die Temperatur des Wassers oder Dampfes sollte zwischen 110 - 500°C, vorzugsweise 150 - 400°C, besonders bevorzugt 180°C - 300°C betragen. Durch die hohe Temperatur des Wassers oder Dampfes und die turbulente Mischung wird die Suspension des Wirkstoffes in der Polymerlösung in kürzester Zeit auf Temperaturen oberhalb von 100°C erhitzt und der Wirkstoff geht in Lösung. Direkt im Anschluß an die Passage der Mischzelle erfolgen die Zerstäubung in einer Sprühdüse und die Sprühtrocknung. Die Temperatur der zu versprühenden Lösung wird geregelt durch die Temperaturen der beiden Flüssigkeitsströme und das Mischungsverhältnis derselben. Höhere Temperaturen des Wasser- oder Dampfstromes und eine größeres Verhältnis Wasser- oder Dampfstrom zu Wirkstoff-Polymersuspension erhöhen die Temperatur der zu versprühenden Wirkstofflösung. Die Verweilzeit in der Mischzelle hängt von der Strömungsgeschwindigkeit der beiden Flüssigkeitsströme sowie der Geometrie der Mischzelle ab. In der Regel wird die Suspension des Wirkstoffes in der Polymerlösung innerhalb von Sekundenbruchteilen auf die gewünschte Temperatur gebracht. Die Temperaturbelastung des Wirkstoffes ist ferner davon abhängig wie schnell im Anschluss an die Mischung die Sprühtrocknung erfolgt. Die Strecke zwischen Mischzelle und Sprühdüse sollte daher entsprechend klein sein. Zur Auflösung der Wirkstoffkristalle ist eine Mindestverweilzeit erforderlich, die aus der wirkstoffspezifischen Auflösungsgeschwindigkeit, der Temperatur der Lösung bzw. Suspension und der Teilchengröße resultiert. Die Gesamtverweilzeit des Wirkstoffes bei hohen Temperaturen kann eingestellt werden durch die Strömungsgeschwindigkeit, die Geometrie der Mischzelle und der Länge der Strecke bis zur Sprühdüse. In der Regel beträgt die Gesamtverweilzeit kleiner 30 Sekunden, vorzugsweise kleiner 15 Sekunden und besonders bevorzugt kleiner 5 Sekunden.

Bei hoher Auflösungsgeschwindigkeit des Wirkstoffes können auch Zeiten kleiner 1 Sekunde erzielt werden.

Die Volumenströme können im Verhältnis 9 : 1 bis 1 : 9 variiert werden.

Die Geometrie der Mischzelle kann sehr vielfältig gestaltet sein. Von einem einfachen T-Stück bis zu sehr ausgefeilten, hochturbulent mischenden Zellen. Der Winkel mit dem die Ströme zusammengeführt werden, kann zwischen 5 und 180° betragen. In einer besonderen Ausführung kann ein Strom mittels einer Injektordüse in den anderen Strom injiziert werden.

Weitere Hilfsstoffe wie zum Beispiel Solubilisatoren werden in der Regel in den wirkstoffhaltigen Strom eingebracht, jedoch können sie prinzipiell auch über die heiße Wasserphase eingeschleust werden.

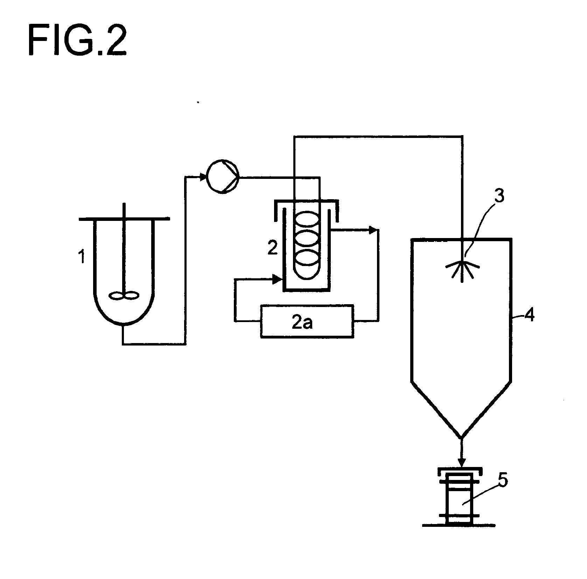

Eine solche Vorgehensweise ist in Abbildung **2** schematisch dargestellt. Dabei wird in einem mit einem Rührer ausgestatteten Behälter 6 eine Wirkstoffsuspension in einer Lösung der Matrixhilfsstoffe hergestellt und kontinuierlich in eine Mischzelle 8 gepumpt. Wasser wird aus einem Behälter 7 kontinuierlich durch einen Wärmetauscher 7a gepumpt, der mit einer Heizung 7b versehen ist, und als überhitztes Wasser oder Dampf ebenfalls in die Mischzelle 8 gepumpt. In der Mischzelfe 8 erfolgt durch kontinuierliches Vermischen der beiden Ströme das Erhitzen und die Auflösung des Wirkstoffs. Die heisse Lösung wird dann über eine Düse 9 in einem Sprühturm 10 zerstäubt und die teilchenförmige feste Lösung 11 aufgefangen.

Das nach dem erfindungsgemäßen Verfahren hergestellte Pulver weist aufgrund seiner Porosität sehr gute Tablettiereigenschaften auf. Üblicherweisen werden mittlere Korngrößen von 25 bis 500 µm erhalten.

Der Vorteil der erfindungsgemäß hergestellten Zubereitungen liegt darin, dass hohe Konzentrationen an Wirkstoff als feste, molekulardisperse Lösung vorliegen, sodass sich die feste Lösung in wässrigem Milieu schnell auflöst und der Wirkstoff im wässrigen Milieu lange Zeit im übersättigten Bereich gehalten wird. Dadurch wird eine hohe biologische Wirkung erzielt.

Die erfindungsgemäß hergestteellten Zubereitungen weisen eine hervorragende Tablettierbarkeit auf, die wesentlich besser ist als bei Anwendung bisher bekannter Herstellungsverfahren. Es resultieren Tabletten mit hoher Bruchfestigkeit und sehr niedriger Friabilität. In der Regel sind die erfindungsgemäßen Zubereitungen direkttablettierbar.

Durch die Zugabe eines Retardierungsmittels kann die Wirkstofffreisetzung entsprechend gesteuert werden. In idealer Weise lassen sich somit Retardformen von schwerlöslichen Wirkstoffen herstellen, die eine sehr reproduzierbare Freisetzung aufweisen.

### Beispiele

### Beispiel 1

### Feste Lösung von Theophyllin in Povidon

10,0 kg Kollidon 30 wurden in 40,0 kg demineralisiertem Wasser gelöst. In dieser Polymerlösung wurden 5,0 kg feingemahlenes Theophyllin unter starkem Rühren suspendiert. Die Kurzzeithocherhitzung erfolgte durch Hindurchpumpen der Lösung durch eine dünne spiralförmige Rohrleitung mit einem Durchmesser von 10 mm, die sich in einem Ölbad mit einer Temperatur von 155 °C befand, wobei die Temperatur der Lösung auf 150°C anstieg. Die Durchflussgeschwindigkeit, die mit einer Hochdruckpumpe eingestellt wurde, betrug 800 - 1000 ml/min. Der Druck in der Rohrleitung betrug 10,45 MPa. Diese heiße Lösung wurde über eine Einstoffdüse mit 0,6 mm Durchmesser bei einem Druck von 100 bar in einem Sprühtrockner zerstäubt. Bei einer Zulufttemperatur von 145°C stellte sich eine Ablufttemperatur von 95°C ein. Es wurde ein trockenes, rieselfähiges Pulver erhalten.

### Beispiel 2

### Feste Lösung von Carbamazepin in Povidon

10,0 kg Kollidon 30 wurden in 40,0 kg demineralisiertem Wasser gelöst. In dieser Polymerlösung wurden 5,0 kg feingemahlenes Carbamazepin unter starkem Rühren suspendiert. Die Kurzzeithocherhitzung erfolgte durch Hindurchpumpen der Lösung durch eine dünne spiralförmige Rohrleitung mit einem Durchmesser von 10 mm, die sich in einem Ölbad mit einer Temperatur von 130°C befand, wobei die Temperatur der Lösung auf 125°C anstieg. Die Durchflussgeschwindigkeit, die mit einer Hochdruckpumpe eingestellt wurde, betrug 700 - 800 ml/min, bei einem Druck von 9 MPa. Diese heiße Lösung wurde über eine Einstoffdüse mit 0,7 mm Durchmesser bei einem Druck von 90 bar in einem FSD-Sprühtrockner zerstäubt und agglomeriert. Bei einer Zulufttemperatur von 145°C stellte sich eine Ablufttemperatur von 96°C ein. Es wurde ein trockenes Pulver mit ausgezeichneten Fließeigenschaften erhalten.

### Beispiel 3

### Feste Lösung von Sulfathiazol in Copolyvidon

10,0 kg Kollidon VA 64 und 1,0 kg Mannit wurden in 40,0 kg demineralisiertem Wasser gelöst. In dieser Polymerlösung wurden 5,0 kg feingemahlenes Sulfathiazol unter starkem Rühren suspendiert. Die Kurzzeithocherhitzung erfolgte durch Hindurchpumpen der Lösung durch eine dünne spiralförmige Rohrleitung mit einem Durchmesser von 10 mm, die sich in einem Ölbad mit einer Temperatur von 130 °C befand, wobei die Temperatur der Lösung auf 116°C anstieg. Die Durchflussgeschwindigkeit, die mit einer Hochdruckpumpe eingestellt wurde, betrug 600 - 800 ml/min, bei einem Druck von 9,1 MPa. Diese heiße Lösung wurde über eine Einstoffdüse mit 0,6 mm Durchmesser bei einem Druck von 90 bar in einem Sprühtrockner zerstäubt. Bei einer Zulufttemperatur von 115°C stellte sich eine Ablufttemperatur von 55°C ein. Es wurde ein trockenes, rieselfähiges Pulver erhalten.

### Beispiel 4

### Feste Lösung von Piroxicam in Copolyvidon/Povidon 1:1

4,5 kg Kollidon VA 64, 5,0 kg Kollidon 30, 0,25kg Nicotinamid und 0,5 kg Natriumlaurylsulfat wurden in 40,0 kg demineralisiertem Wasser gelöst. In dieser Polymerlösung wurden 3,0 kg feingemahlenes Piroxicam unter starkem Rühren suspendiert. Die Kurzzeithocherhitzung erfolgte durch Hindurchpumpen der Lösung durch eine dünne spiralförmige Rohrleitung mit einem Durchmesser von 10 mm, die sich in einem Ölbad mit einer Temperatur von 200°C befand, wobei die Temperatur der Lösung auf 160°C anstieg. Die Durchflussgeschwindigkeit, die mit einer Hochdruckpumpe eingestellt wurde, betrug 600 - 700 ml/min, bei einem Druck von 9,1 MPa. Diese heiße Lösung wurde über eine Einstoffdüse mit 0,6 mm Durchmesser bei einem Druck von 90 bar in einem Sprühtrockner zerstäubt. Bei einer Zulufttemperatur von 125°C stellte sich eine Ablufttemperatur von 70°C ein. Es wurde ein trockenes, rieselfähiges Pulver erhalten.

### Beispiel 5

### Feste Lösung von Clotrimazol in Povidon K 17

10,0 kg Kollidon 17 PF, 0,3kg Lutrol F 68 (Poloxamer 188) und 0,3kg Natriumstearat wurden in 40,0 kg demineralisiertem Wasser gelöst. In dieser Polymerlösung wurden 2,0 kg feingemahlenes Clotrimazol unter starkem Rühren suspendiert. Die Kurzzeithocherhitzung erfolgte durch Hindurchpumpen der Lösung durch eine dünne spiralförmige Rohrleitung mit einem Durchmesser von 10 mm, die sich in einem Ölbad mit einer Temperatur von 135°C befand, wobei die Temperatur der Lösung auf 115°C anstieg. Diese heiße Lösung wurde mit einer Zweistoffdüse in einem Sprühwirbelschichttrockner zerstäubt. Bei einer Zulufttemperatur von 100°C stellte sich eine Ablufttemperatur von 65°C ein. Es wurde ein trockenes, relativ feines, gut rieselfähiges Pulver erhalten.

### Beispiel 6

### Feste Lösung von Cinnarizin in Povidon

10,0 kg Kollidon 30 und 0,5kg Cremophor RH 40 (Umsetzungsprodukt von hydriertem Rizinusöl mit 45 mol Ethylenoxid) wurden in 40,0 kg demineralisiertem Wasser gelöst. In dieser Polymerlösung wurden 2,5 kg Cinnarizin unter starkem Rühren suspendiert und anschließend durch Behandlung mit einem Ultra-turrax in 15min homogenisiert. Ein Strom dieser feinteiligen Suspension wurde über einen Wärmetauscher auf 60°C erwärmt und über ein T-Stück mit einem Strom 280°C heißen Wassers vereinigt. Das Verhältnis des wirkstoffhaltigen Stromes zum heißen Wasserstrom betrug 1:2. Die Temperatur der heißen Lösung vor der Sprühdüse betrug 195°C und die Verweilzeit bei dieser Temperatur 2,5 Sekunden. Diese heiße Lösung wurde über eine Einstoffdüse mit 0,6 mm Durchmesser bei einem Druck von 100 bar in einem Sprühtrockner zerstäubt. Bei einer Zulufttemperatur von 145°C stellte sich eine Ablufttemperatur von 95°C ein. Es wurde ein trockenes, rieselfähiges Pulver erhalten.

### Beispiel 7

### Feste Lösung von Ketoconazol in Povidon/Polyvinylcaprolactam

8,0 kg Kollidon 30, 2,0kg Polyvinylcaprolactam K-Wert 30 und 0,2kg Polysorbat 80 und 0,1kg Ascorbylpalmitat wurden in 40,0 kg demineralisiertem Wasser gelöst. In dieser Polymerlösung wurden 2,0 kg Ketoconazol unter starkem Rühren suspendiert und anschließend durch Behandlung mit einem Ultra-turrax in 15min homogenisiert. Ein Strom dieser feinteiligen Suspension wurde über einen Wärmetauscher auf 50°C erwärmt und über eine Mischzelle mit einem Strom 240°C heißen Wassers vereinigt. Das Verhältnis des wirkstoffhaltigen Stromes zum heißen Wasserstrom betrug 1:5. Die Temperatur der heißen Lösung vor der Sprühdüse betrug 200°C und die Verweilzeit bei dieser Temperatur 2,0 Sekunden. Diese heiße Lösung wurde über eine Einstoffdüse mit 0,6 mm Durchmesser bei einem Druck von 100 bar in einem Sprühtrockner zerstäubt. Bei einer Zulufttemperatur von 145°C stellte sich eine Ablufttemperatur von 95°C ein. Es wurde ein trockenes, rieselfähiges Pulver erhalten.

### Beispiel 8

### Feste Lösung von Indometacin in Povidon

10,0 kg Kollidon 30, 0,2kg Polyethylenglykol 6000 und 0,2kg Cremophor RH 40 wurden in 40,0 kg demineralisiertem Wasser gelöst. In dieser Polymerlösung wurden 2,0 kg Indometacin unter starkem Rühren suspendiert und anschließend durch Behandlung mit einem Ultra-turrax in 15min homogenisiert. Ein Strom dieser feinteiligen Suspension wurde über einen Wärmetauscher auf 50°C erwärmt und über eine Mischzelle mit einem Strom 280°C heißen Wassers vereinigt. Das Verhältnis des wirkstoffhaltigen Stromes zum heißen Wasserstrom betrug 1:1. Die Temperatur der heißen Lösung vor der Sprühdüse betrug 158°C und die Verweilzeit bei dieser Temperatur 1,5 Sekunden. Diese heiße Lösung wurde über eine Einstoffdüse mit 0,6 mm Durchmesser bei einem Druck von 100 bar in einem Sprühtrockner zerstäubt. Gleichzeitig wurde über eine separate Düse Aerosil 200 in den Turm eingestäubt, wobei das Verhältnis Feststoff aus der Lösung zu Aerosil 99:1 betrug. Bei einer Zulufttemperatur von 143°C stellte sich eine Ablufttemperatur von 82°C ein. Es wurde ein trockenes, rieselfähiges Pulver erhalten.

### Beispiel 9

### Feste Lösung von Betacarotin in Povidon

8,0 kg Kollidon 25, 1,5kg Cremophor RH 40, 0,5 kg Harnstoff, 0,1kg Ascorbylpalmitat und 0,05kg Butylhydroxytoluol wurden in 30,0 kg sauerstofffreiem, demineralisiertem Wasser gelöst. In dieser Polymerlösung wurden 1,0 kg Betacarotin unter starkem Rühren suspendiert und anschließend durch Behandlung mit einem Ultra-turrax in 15min homogenisiert. Ein Strom dieser feinteiligen Suspension wurde über einen Wärmetauscher auf 70°C erwärmt und über eine Mischzelle mit einem Strom 230°C heißen Wassers vereinigt. Das Verhältnis des wirkstoffhaltigen Stromes zum heißen Wasserstrom betrug 1:3. Die Temperatur der heißen Lösung vor der Sprühdüse betrug 190°C und die Verweilzeit bei dieser Temperatur 3,0 Sekunden. Diese heiße Lösung wurde über eine Einstoffdüse mit 0,7 mm Durchmesser bei einem Druck von 100 bar in einem Sprühtrockner zerstäubt. Bei einer Zulufttemperatur von 120°C stellte sich eine Ablufttemperatur von 69°C ein. Es wurde ein trockenes, rieselfähiges Pulver erhalten.

### Beispiel 10

### Feste Lösung von Theophyllin in Harnstoff

10,0 kg Harnstoff wurden in 40,0 kg demineralisiertem Wasser gelöst. In dieser Polymerlösung wurden 3,0 kg feingemahlenes Theophyllin unter starkem Rühren suspendiert. Die Kurzzeithocherhitzung erfolgte durch Hindurchpumpen der Lösung durch eine dünne spiralförmige Rohrleitung mit einem Durchmesser von 3 mm, die sich in einem Ölbad mit einer Temperatur von 155°C befand, wobei die Temperatur der Lösung auf 150°C anstieg. Die Durchflussgeschwindigkeit, die mit einer Hochdruckpumpe eingestellt wurde, betrug 500 - 600 ml/min. Diese heiße Lösung wurde über eine Einstoffdüse mit 0,5 mm Durchmesser bei einem Druck von 100 bar in einem Sprühtrockner zerstäubt. Bei einer Zulufttemperatur von 125°C stellte sich eine Ablufttemperatur von 77°C ein. Es wurde ein trockenes, rieselfähiges Pulver erhalten.

### Vergleichsbeispiel 1

### Feste Dispersion von Theophyllin in Povidon

10,0 kg Kollidon 30 wurden in 40,0 kg demineralisiertem Wasser gelöst. In dieser Polymerlösung wurden 5,0 kg Theophyllin unter starkem Rühren suspendiert. Diese Suspension wurde über eine Einstoffdüse mit 0,5mm Durchmesser bei einem Druck von 100bar in einem Sprühtrockner zerstäubt. Bei einer Zulufttemperatur von 160°C stellte sich eine Ablufttemperatur von 90°C ein. Es wurde ein trockenes, rieselfähiges Pulver erhalten.

### Vergleichsbeispiel 2

### Feste Dispersion von Carbamazepin in Povidon

10,0 kg Kollidon 30 wurden in 40,0 kg demineralisiertem Wasser gelöst. In dieser Polymerlösung wurden 5,0 kg Carbamazepin unter starkem Rühren suspendiert. Diese Suspension wurde über einer Zweistoffdüse in einem FSD-Sprühtrockner zerstäubt und agglomeriert. Bei einer Zulufttemperatur von 155°C stellte sich eine Ablufttemperatur von 88°C ein. Es wurde ein trockenes Pulver erhalten.

**Tabelle 1**

| **Beispiele** | **Mikroskopische Beurteilung** | **DSC-Untersuchung** | **Röntgendiffraktometrie** |
|---|---|---|---|
| Beispiel 1 Theophyllin | keine Kristalle | Kein Wirkstoffpeak bei 270-274°C | amorph |
| Beispiel 2 Carbamazepin | keine Kristalle | Kein Wirkstoffpeak bei 191 °C | amorph |
| Beispiel 3 Sulfathiazol | keine Kristalle | Kein Wirkstoffpeak bei 202°C | amorph |
| Beispiel 4 Piroxicam | keine Kristalle | Kein Wirkstoffpeak bei 205°C | amorph |
| Beispiel 5 Clotrimazol | keine Kristalle | Kein Wirkstoffpeak bei 148°C | amorph |
| Beispiel 6 Cinnarizin | keine Kristalle | Kein Wirkstoffpeak | amorph |
| Beispiel 7 Ketoconazol | keine Kristalle | Kein Wirkstoffpeak bei 146°C | amorph |
| Beispiel 8 Indometacin | keine Kristalle | Kein Wirkstoffpeak bei 155°C | amorph |
| Beispiel 9 Betacarotin | keine Kristalle | Kein Wirkstoffpeak | amorph |
| Beispiel 10 Theophyllin | keine Kristalle | Kein Wirkstoffpeak bei 270-274°C | amorph |
| Vergleichsbeispiel zu 1 | Kristalle | Wirkstoffpeak bei 270-274°C | Kristalline Anteile |
| Vergleichsbeispiel zu 2 | Kristalle | Wirkstoffpeak bei 191°C | Kristalline Anteile |

### Beispiel 11

### Theophyllin Tabletten

2,1 kg Theophyllin feste Lösung aus Beispiel 1 wurden mit 1,5 kg Ludipress® LCE (coprozessiertes Produkt aus 93% Lactose, 3,5 % Povidon, 3,5 % Crospovidon) ; 0,03kg hochdisperse Kieselsäure (Aerosil 200, Fa. Degussa), 0,15kg Crospovidon (Kollidon CL, Fa. BASF) und 0,03kg Magnesiumstearat in einem Turbulamischer 10 min gemischt und mit einer Rundläufertablettenpresse des Typs Korsch PH 106 bei 18kN Presskraft zu Tabletten gepresst. Die Tabletten hatten einen Durchmesser von 10mm und ein Gewicht von 331mg.

Analog wurden auch Tabletten mit dem Pulver aus dem Vergleichsbeispiel 1 sowie mit reinen Theophyllin Kristallen gepresst.

Von den Tabletten wurden die Bruchfestigkeit und die Wirkstofffreisetzung in einer Paddle Apparatur nach USP 2004 bestimmt.

| Produkt | Bruchfestigkeit | Freisetzung nach 15min in % | Freisetzung nach 30min in % |
|---|---|---|---|
| Beispiel 1 | 225 | 62 | 99 |
| Vergleichsbeispiel 1 | 174 | 42 | 80 |
| Theophyllinkristalle | 140 | 34 | 78 |

### Beispiel 14

### Carbamazepin Kapseln

180g Carbamazepin feste Lösung gemäß Beispiel 2 wurden mit 100g Calciumhydrogenphosphat, 1,5g Aerosil 200 und 20g Kollidon CL in einem Turbulamischer 10 min gemischt und in Gelatinekapseln gefüllt, wobei die Füllmenge 301,5mg betrug.

Analog wurden auch Kapseln mit dem Pulver aus dem Vergleichsbeispiel 2 sowie mit reinen Carbamazepin Kristallen hergestellt.

Von den Kapseln wurde die Wirkstofffreisetzung in einer Paddle Apparatur nach USP 2004 bestimmt.

| Produkt | Freisetzung nach 15min in % | Freisetzung nach 30min in % |
|---|---|---|
| Beispiel 2 | 55 | 101 |
| Vergleichsbeispiel 2 | 35 | 66 |
| Carbamazepinkristalle | 22 | 53 |

## Patentansprüche

1. Verfahren zur Herstellung von pulver- oder granulatförmigen festen Lösungen schwerlöslicher Substanzen, wobei die Sättigungslöslichkeit der schwerlöslichen Substanzen bei 20 °C in mindestens einem der Medien Wasser, 0,1 molare Salzsäure, wässriger Phospatpufffer pH 7,2 oder 0.9 gew.-%iger Kochsalhlösung kleiner als 1 Gew.-% ist, in denen die schwerlösliche Substanz molekulardispers in einer Hilfsstoffmatrix vorliegt, wobei der molekulardisperse Zustand mit Hilfe der Methode der Differential Scanning Calorimetry derart ermittelt werden kann, dass im Bereich des Schmelzpunkts des Wirkstoffs kein Schmelzpeak mehr zu beobachten ist, durch Zerstäuben einer Lösung des Wirkstoff und der Matrixhilfsstoffe, **dadurch gekennzeichnet, dass** eine wässrige Suspension der schwerlöslichen Substanz in Gegenwart der Matrixhilfsstoffe auf Temperaturen oberhalb der Siedetemperatur bei Normaldruck erhitzt und die schwerlösliche Substanz in Lösung gebracht wird, und die Lösung der schwerlöslichen Substanz und der Matrixhilfsstoffe anschließend durch Zerstäuben und Trocknen in eine feste Form überführt wird, wobei die Temperatur der Sprühlösung vor der Zuführung in die Zerstäubungsvorrichtung 90°C bis 350 °C beträgt.

2. Verfahren nach Anspruch 1 **dadurch gekennzeichnet, dass** die Temperatur der Sprühlösung vor der Zerstäubung 110 - 300°C beträgt und der Wirkstoff gelöst vorliegt.

3. Verfahren nach Anspruch 1 **dadurch gekennzeichnet, dass** die Temperatur der Sprühlösung vor der Zerstäubung 120 - 250°C beträgt und der Wirkstoff gelöst vorliegt.

4. Verfahren nach einem der Ansprüche 1 bis 3, **dadurch gekennzeichnet, dass** die Verweilzeit des Wirkstoffes bei Temperaturen über 90°C kleiner 180 Sekunden beträgt.

5. Verfahren nach einem der Ansprüche 1 bis 3,**dadurch gekennzeichnet, dass** die Verweilzeit des Wirkstoffes bei Temperaturen über 90°C kleiner 60 Sekunden beträgt.

6. Verfahren nach einem der Ansprüche 1 bis 3, **dadurch gekennzeichnet, dass** die Verweilzeit des Wirkstoffes bei Temperaturen über 90°C kleiner 15 Sekunden beträgt.

7. Verfahren nach einem der Ansprüche 1 bis 6, **dadurch gekennzeichnet, dass** die Konzentration der schwerlöslichen Substanz in der Hilfsstoffmatrix 1 bis 50 Gew.-%, bevorzugt größer 10% und besonders bevorzugt größer 20% beträgt.

8. Verfahren nach einem der Ansprüche 1 bis 7, **dadurch gekennzeichnet, dass** die Konzentration der schwerlöslichen Substanz in der Hilfsstoffmatrix 10 bis 50 Gew.-%, beträgt.

9. Verfahren nach einem der Ansprüche 1 bis 8, **dadurch gekennzeichnet, dass** die Konzentration der schwerlöslichen Substanz in der Hilfsstoffmatrix 20 bis 50 Gew.-% beträgt.

10. Verfahren nach einem der Ansprüche 1 bis 9, **dadurch gekennzeichnet, dass** zur Bildung der festen Lösung Matrixhilfsstoffe mit Amidgruppen eingesetzt werden.

11. Verfahren nach einem der Ansprüche 1 bis 10, **dadurch gekennzeichnet, dass** als Matrixhilfsstoffe zur Bildung der festen Lösung Homo- oder Copolymere von N-Vinylpyrrolidon, N-Vinylcaprolactam, N-Vinylformamid oder N-Vinylacetamid eingesetzt werden.

12. Verfahren nach einem der Ansprüche 1 bis 11, **dadurch gekennzeichnet, dass** die festen Lösungen zusätzlich als Solubilisatoren Tenside mit einem HLB-Wert größer 10enthalten.

13. Verfahren nach einem der Ansprüche 1 bis 12, **dadurch gekennzeichnet, dass** die festen Lösungen zusätzlich Cosolubilisatoren mit einem HLB-Wert kleiner 10 enthalten.

14. Verfahren nach einem der Ansprüche 1 bis 13, **dadurch gekennzeichnet, dass** die Sprühlösung ein Adsorbens enthält.

15. Verfahren nach einem der Ansprüche 1 bis 14, **dadurch gekennzeichnet, dass** die Erhitzung der Suspension des Wirkstoffes über einen Wärmeaustauscher erfolgt.

16. Verfahren nach einem der Ansprüche 1 bis 15, **dadurch gekennzeichnet, dass** die Erhitzung der Suspension des Wirkstoffes durch Vermischen mit einem heißen Flüssigkeitsstrom oder einem heißem Dampfstrom erfolgt.

17. Verfahren nach Anspruch 16, **dadurch gekennzeichnet, dass** das Verhältnis der Suspension des Wirkstoffes zum heißen Flüssigkeitsstrom zwischen 9 : 1 und 1 : 9 liegt.

18. Verfahren nach einem der Ansprüche 16 oder 17, **dadurch gekennzeichnet, dass** das Verhältnis der Suspension des Wirkstoffes zum heißen Flüssigkeitsstrom zwischen 7 : 3 und 3 : 7 liegt.

19. Verfahren nach einem der Ansprüche 16 bis 18, **dadurch gekennzeichnet, dass** die Temperatur des heißen Flüssigkeits- oder Dampfstromes zwischen 110 - 500°C beträgt.

20. Verfahren nach einem der Ansprüche 16 bis 19, **dadurch gekennzeichnet, dass** die Temperatur des heißen Flüssigkeits- oder Dampfstromes zwischen 150 - 400°C beträgt.

21. Verfahren nach einem der Ansprüche 16 bis 20, **dadurch gekennzeichnet, dass** die Temperatur des heißen Flüssigkeits- oder Dampfstromes zwischen 180°C - 300°C beträgt.

22. Verfahren nach einem der Ansprüche 1 bis 21, **dadurch gekennzeichnet, dass** zur Trocknung Verfahren der Zerstäubungstrocknung oder der Wirbelschicht-Sprühgranulation verwendet werden.

23. Verfahren nach einem der Ansprüche 1 bis 22, **dadurch gekennzeichnet, dass** während der Zerstäubungstrocknung ein Adsorbens oder Puderungsmittel in den Sprühturm eingeblasen wird.

24. Verfahren nach einem der Ansprüche 1 bis 23, **dadurch gekennzeichnet, dass** es sich bei den schwerlöslichen Wirkstoffen um Arzneistoffe, Vitamine, Carotinoide oder Nutraceuticals handelt.

## Claims

1. A process for producing solid solutions in powder or granule form of slightly soluble substances, where the saturation solubility of the slightly soluble substances at 20°C in at least one of the media waster, 0.1 molar hydrochloric gas, aqueous phosphate buffer pH 7.2 or 0.9% by weight strength sodium chloride solution is less than 1% by weight, in which the slightly soluble substance is in the form of a molecular dispersion in an excipient matrix, where the molecularly dispersed state can be determines by differential scanning calorimetry such that in the range of the melting point of the active compound no melting peak can be observed, by atomizing a solution of the active ingredient and of the matrix excipients, which comprises heating an aqueous suspension of the slightly soluble substance in the presence of the matrix excipients to temperatures above the boiling paint under atmospheric pressure, and dissolving the slightly soluble substance, and subsequently converting the solution of the slightly soluble substance and of the matrix excipients by atomizing and drying into a solid form, where the temperature of the spray solution before feeding into the atomizing apparatus is 90°C to 350°C.

2. The process according to claim 1, wherein tie temperature of the spray solution before the atomizing is 110-300°C, and the active ingredient is in dissolved form.

3. The process according to claim 1, wherein the temperature of the spray solution before the atomizing is 120-250°C, and the active ingredient is in dissolved form.

4. The process according to any of claims 1 to 3, wherein the residence time of the active ingredient at temperatures above 90°C is less than 180 seconds.

5. The process according to any of claims 1 to 3, wherein the residence time of the active ingredient at temperatures above 90°C is less than 60 seconds.

6. The process according to any of claims 1 to 3, wherein the residence time of the active ingredient at temperatures above 90°C is less than 15 seconds.

7. The process according to any of claim 1 to 6, wherein the concentration of the slightly soluble substance in the excipient matrix is 1 to 50% by weight, preferably above 10% and particularly preferably above 20%.

8. The process according to any of claims 1 to 7, wherein the concentration of the slightly soluble substance in the excipient matrix is from 10 to 50% by weight.

9. The process according to any of claims 1 to 8, wherein the concentration of the slightly soluble substance in the excipient matrix is from 20 to 50% by weight.

10. The process according to any of claims 1 to 9, wherein matrix excipients with amide groups are employed to form the solid solution.

11. The process according to any of claims 1 to 10, wherein the matrix excipients employed to form the solid solution are homo- or copolymers of N-vinylpyrrolidone, N-vinylcaprolactam, N-vinylformamide or N-vinylacetamide.

12. The process according to any of claims 1 to 11, wherein the solid solutions additionally comprise surfactants having a HLB above 10 as solubilizers.

13. The process according to any of claims 1 to 12, wherein the solid solutions additionally comprise cosolubilizers having a HLB bellow 10.

14. The process according to any of claims 1 to 13, wherein the spray solution comprises an adsorbent.

15. The process according to any of claims 1 to 14, wherein the suspension of the active ingredient is heated by a heat exchanger.

16. The process according to any of claims 1 to 15, wherein the heating of the suspension of the active ingredient takes place by fixing with a hot stream of liquid or a hot stream of vapor.

17. The process according to claim 16, wherein the ratio of the suspension of the active ingredient to the hot stream of liquid is between 9:1 and 1:9.

18. The process according to either of claims 16 or 17, wherein the ratio of the suspension of the active ingredient to the hot stream of liquid is between 7:3 and 3:7.

19. The process according to any of claims 16 to 18, wherein the temperature of the hot stream of liquid or vapor is between 110-500°C.

20. The process according to any of claims 16 to 19, wherein the temperature of the hot stream of liquid or vapor is between 150-400°C.

21. The process according to any of claims 16 to 20, wherein the temperature of the hot stream of liquid or vapor is between 180-300°C.

22. The process according to any of claims 1 to 21, wherein processes of atomizing drying or of fluidized bed spray granulation are used for the drying.

23. The process according to any of claims 1 to 22, wherein an adsorbant or dusting agent is blown into the spray tower during the atomizing drying.

24. The process according to any of claim 1 to 23, wherein the slightly soluble active ingredients are medicinal substance, vitamins, carotenoids or nutraceuticals.

## Revendications

1. Procédé pour la préparation de solutions solides, sous forme de poudre ou de granulat, de substances peu solubles, la solubilité de saturation des substances peu solubles à 20°C dans au moins un des milieux eau, acide chlorhydrique à 0,1 M, tampon phosphate aqueux pH 7,2 ou solution de chlorure de sodium à 0,9% en poids étant inférieure à 1% en poids, dans lesquelles la substance peu soluble se trouve sous forme de dispersion moléculaire dams une matrice d'adjuvants, l'état de dispersion moléculaire pouvant être déterminé à l'aide du procédé de calorimétrie différentielle à balayage de manière telle que dans la plage du point de fusion de la substance active, on n'observe plus de pic de fusion, par valorisation d'une solution de la substance active et des adjuvants de matrice, **caractérisé en ce qu'**on chauffe une suspension aqueuse de la substance peu soluble en présence des adjuvants de matrice à des températures supérieures à la température d'ébullition à pression normale et on amène la substance peu soluble en solution, on transfère ensuite la solution de la substance peu soluble et des adjuvants de matrice par vaporisation et séchage dans un état solide, la température de la solution pulvérisée avant l'introduction dans le dispositif de valorisation étant de 90°C à 350°C.

2. Procédé selon la revendication 1, **caractérisé en ce que** la température de la solution pulvérisée avant la valorisation est de 110-300°C et la substance active se trouve sous forme dissoute.

3. Procédé selon la revendication 1, **caractérisé en ce que** la température de la solution pulvérisée avant la vaporisation est de 120-250°C et la substance active se trouve sous forme dissoute.

4. Procédé selon l'une quelconque des revendication 1 à 3, **caractérisé en ce que** le temps de séjour de la substance active à des températures supérieures à 90°C est inférieur à 180 secondes.

5. Procédé selon l'une quelconque des revendications 1 à 3, **caractérisé en ce que** le temps de séjour de la substance active à des températures supérieures à 90°C est intérieur à 60 secondes.

6. Procédé selon l'une quelconque des revendications 1 à 3, **caractérisé en ce que** le temps de séjour de la substance active à des températures supérieures à 90°C est inférieur à 15 secondes.

7. Procédé selon l'une quelconque des revendications 1 à 6, **caractérisé en ce que** la concentration en substance peu soluble dans la matrice d'adjuvants est de 1 à 50% en poids, de préférence supérieure à 10% et de manière particulièrement préférée supérieure à 20%.

8. Procédé selon l'une quelconque des revendications 1 à 7, **caractérisé en ce que** la concentration en substance peu soluble dans la matrice d'adjuvants est de 10 à 50% en poids.

9. Procédé selon l'une quelconque des revendications 1 à 8, **caractérisé en ce que** la concentration en substance peu soluble dans la matrice d'adjuvants est de 20 à 50% en poids.

10. Procédé selon l'une quelconque des revendications 1 à 9, **caractérisé en ce qu'**on utilise, pour la formation de la solution solide, des adjuvants de matrice présentant des groupes amide.

11. Procédé selon l'une quelconque des revendications 1 à 10, **caractérisé en ce qu'**on utilise comme adjuvants de matrice pour la formation de la solution solides des homopolymères ou des copolymères de N-vinyipyrrolidone, de N-vinylcaprolactame, de N-vinylformamide ou de N-vinylacétamide.

12. Procédé selon l'une quelconque des revendications 1 à 11, **caractérisé en ce que** les solutions solides contiennent en outre, comme agents de solubilisation, des agents tensioactifs présentent une voleur BHL supérieure à 10.

13. Procédé selon l'une quelconque des revendications 1 à 12, **caractérisé en ce que** les solutions solides contiennent en outre des co-agents de solubilisation présentant une valeur BHL intérieure à 10.

14. Procédé selon l'une quelconque des revendications 1 à 13, **caractérisé en ce que** la solution pulvérisée contient un adsorbant.

15. Procédé selon l'une quelconque des revendications 1 à 14, **caractérisé en ce que** le chauffage de la suspension de la substance active a lieu via un échangeur thermique.

16. Procédé selon l'une quelconque des revendications 1 à 15, **caractérisé en ce que** le chauffage de la suspension de la substance active a lieu par mélange avec un flux de liquide chaud ou un flux de vapeur chaud.

17. Procédé selon la revendication 16, **caractérisé en ce que** le rapport de la suspension de la substance active au flux de liquide chaud se situe entre 9:1 et 1:9.

18. Procédé selon l'une quelconque des revendications 16 ou 17, **caractérisé en ce que** le rapport de la suspension de la substance active au flux de liquide chaud se situe entre 7:3 et 3:7.

19. Procédé selon l'une quelconque des revendications 16 à 18, **caractérisé en ce que** la température du flux de liquide ou de vapeur chaud est située entre 110-500°C.

20. Procédé selon l'une quelconque des revendications 16 à 19, **caractérisé en ce que** la température du flux de liquide ou de vapeur chaud est située entre 150-400°C.

21. Procédé selon l'une quelconque des revendications 16 à 20, **caractérisé en ce que** la température du flux de liquide ou de vapeur chaud est située entre 180-300°C.

22. Procédé selon l'une quelconque des revendications 1 à 21, **caractérisé en ce qu'**on utilise pour le séchage des procédés de séchage par vaporisation ou de granulation par pulvérisation en couche tourbillonnante.

23. Procédé selon l'une quelconque des revendications 1 à 22, **caractérisé en ce qu'**on insuffle, pendant le séchage par vaporisation, un adsorbant ou un agent de saupoudrage dans la tour de séchage.

24. Procédé selon l'une quelconque des revendications 1 à 23, **caractérisé en ce qu'**il s'agit, pour les substances actives peu solubles, de médicaments, de vitamines, de caroténoïdes ou de nutraceutiques.
